# EUROPEAN PATENT SPECIFICATION

(11) **EP 0 449 346 B1**
(45) Date of publication and mention of the grant of the patent: **10.05.1995**
(21) Application number: 91200446.2
(22) Date of filing: 01.03.1991
(51) Int. Cl.: C07D 457/02, C07D 457/00, A61K 31/48

(54) **4-Piperidinyl-ergoline derivatives**
4-Piperidinyl-ergolin-Derivate
Dérivés pipéridinyl-4-ergoline

(30) Priority: 27.03.1990 GB 9006772
(43) Date of publication of application: 02.10.1991
(73) Proprietor: PHARMACIA S.p.A., 20152 Milano (IT)
(72) Inventor: Mantegani, Sergio, I-20129 Milan (IT); Bandiera, Tiziano, I-27025 Gambolò (Pavia) (IT); Brambilla, Enzo, I-22066 Mariano Comense (Como) (IT); Buonamici, Metilde, I-20015 Parabiago (Milan) (IT)
(74) Representative: Ferrario, Vittorino

(56) References cited:
- EP-A- 0 126 968
- EP-A- 0 197 241
- EP-A- 0 206 206
- FR-A- 2 577 553

## Description

The invention relates to ergoline derivatives endowed with dopaminergic activity, to a process for their preparation and to the pharmaceutical compositions containing them.

The invention provides ergoline derivatives having the formula I : 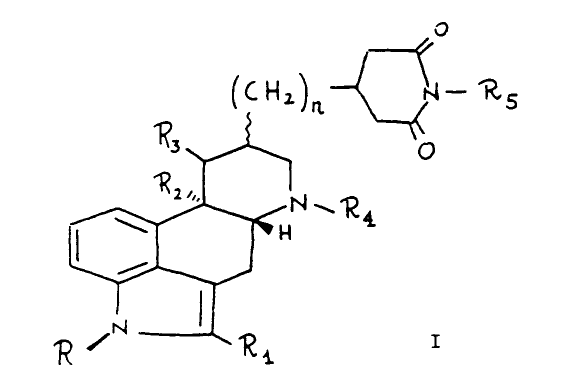
wherein R represents a hydrogen atom or a C₁-C₄ alkyl group, R₁ represents a hydrogen or halogen atom, a methyl or phenylthio group or an alkylthio group having from 1 to 4 carbon atoms; R₂ represents a hydrogen atom or a methoxy group and R₃, represents a hydrogen atom or R₂ and R₃ together represent a chemical bond; R₄ represents a hydrocarbon group having from 1 to 4 carbon atoms; R₅ represents a hydrogen atom, an alkyl group having from 1 to 4 carbon atoms, a phenyl group and n is 0, 1 or 2 and the pharmaceutically acceptable salts thereof.

In formula I, R is preferably methyl or hydrogen. In the definition of R₄, a hydrocarbon group having from 1 to 4 carbon atoms is intended to include alkyl, cycloalkyl and unsaturated (both ethylenically and acetylenically) groups.

R₄ may be methyl, ethyl, n-propyl, iso-propyl, n-butyl, sec-butyl, t-butyl, iso-butyl or allyl.

Preferably R₄ is methyl. When R₁ is halogen, it may be fluorine,
chlorine or bromine. Preferably R₁ is chlorine or bromine or hydrogen.

The wavy line ( 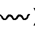 ) in formula I indicates that the substituent in the 8-position may be either in the α-configuration, i.e. below the plane of the ring, or in the β-configuration, i.e. above the plane of the ring.

Preferably the substituent in the 8-position is in the β-configuration.

"Pharmaceutically acceptable salts" refer to those salts which retain the biological effectiveness and properties of the free bases and which are not biologically or otherwise undesiderable.

Preferred ergoline derivatives of the present invention are the following ones :
4-[(6-Methylergolen-Δ9,10-8β-yl)methyl]-piperidin-2,6-dione;
4-[(6-methylergolin-8β-yl)methyl]-piperidin-2,6-dione;
4-[(6-methylergolin-8β-yl)-piperidin-2,6-dione;
4-[(6-Propylergolen-Δ9,10-8β-yl)methyl]-piperidin-2,6-dione;
1-Methyl-4-[(6-methylergolen-Δ9,10-8β-yl)methyl]-piperidin-2,6-dione;
4-[(6-Methylergolen-Δ9,10-8α-yl)methyl]-piperidin-2,6-dione;
1-Phenyl-4-[(6-methylergolen-Δ9,10-8β-yl)methyl]-piperidin-2,6-dione;
4-[(6-Methylergolen-Δ9,10-8β-yl)ethyl]-piperidin-2,6-dione;
4-[(1-Ethyl-6-methylergolen-Δ9,10-8β-yl)methyl]-piperazin-2,6-dione;
4-[(2-Thiomethyl-6-methylergolen-Δ9,10-8β-yl)methyl]-piperidin-2,6-dione.

The invention further comprises a process for the preparation of a compound of the formula I, which comprises reaction of a compound of the formula II : 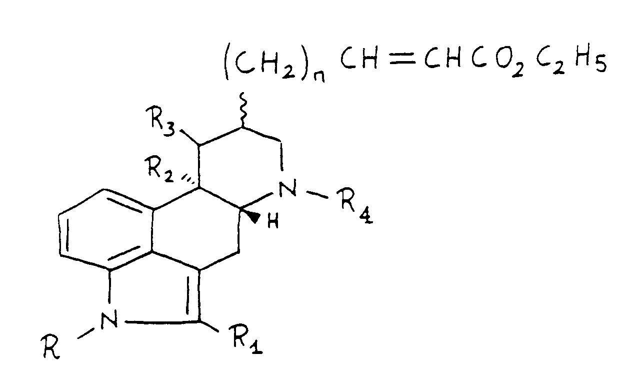
wherein R, R₁, R₂, R₄ and n are as defined above with a compound of the formula III : 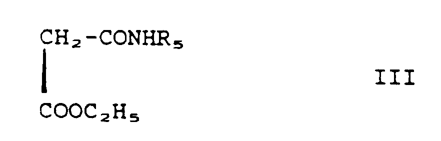
wherein R₅ is as defined above, saponificating and decarboxylating the resultant compound of the formula IV : 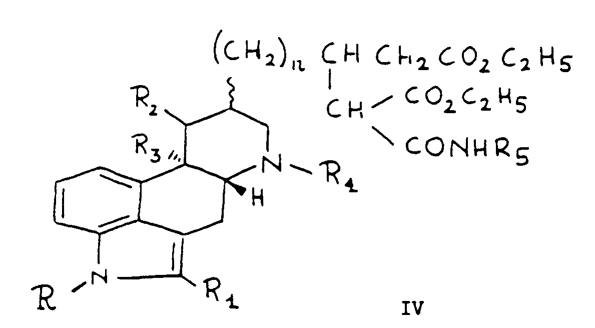
wherein R, R₁, R₂, R₃, R₄, R₅ and n are as defined above and cyclizing the resultant compounds of the formula V : 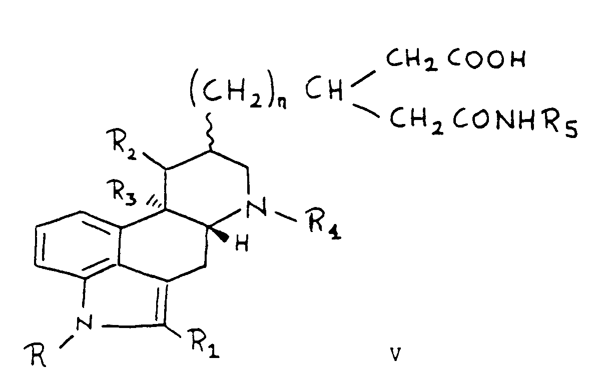
wherein, R, R₁, R₂, R₃, R₄, R₅ and n are as defined above to give the desired compounds of the formula I.

The reaction between the compounds of the formula II and III may be carried out in an inert solvent such as tetrahydrofurane or dioxane, at a temperature of 60°-110° C for some hours optionally in presence of catalytic amount of sodium ethoxide.

The saponification of the compounds of the formula IV is preferably carried out in alkaline conditions, and the subsequent decarboxylation by heating in a suitable solvent such as dimethylformamide or dimethylsulfoxide.

The cyclization of the compounds of the formula V is preferably carried out in presence of a condensing agent such as acetic anhydride or diimidazole carbonyl in a solvent such as tetrahydrofurane, dioxane or acetonitrile at a temperature ranging from 50° C to 110 ° C for some minutes. The final compounds of the formula I may be then purified by known procedures, usually chromatography or crystallization from a suitable solvent.

The preparation of the sarting compounds of formula II is described in U.S.Patent 4,746,666, the compounds of the formula III are known compounds or may be prepared by known procedures.

The ergoline derivatives according to the present invention and their pharmaceutically acceptable salts are effective in the central nervous system (CNS) and are particularly useful for treating extrapyramidal syndromes.

More in particular, the ergoline derivatives according to the invention are active as antiparkinson agents, owing to their dopaminergic activity. The dopaminergic activity is indicated e.g. by an induction of controlateral turnings in rats with unilateral 6-hydroxy dopamine-induced lesion of the dopaminergic nigrostriatal patway. [According to the principles of N.Ungerstedt et al., Brain Research 24,485 (1970)].

The products according to the present invention have a surprisingly higher activity as compared with the known standard reference drug, bromocriptine, as the following table shows :

| EFFECTS OF A SINGLE TREATMENT ON TURNING BEHAVIOUR IN RATS | | | | |
|---|---|---|---|---|
| Product of Example | Dose mg/Kg | Turning rats Treated rats | Number of controlateral turns (X) in 6 hours | Route of administration |
| 1 | 0.5 | 4/4 | 1540 | s.c. |
| 5 | 0.5 | 5/5 | 1830 | s.c. |
| Bromocriptine | 1 | 6/9 | 1920 | s.c. |

Accordingly, the present invention provides a pharmaceutical composition for treating extrapyramidal syndromes such as Parkinson's disease, containing a novel ergoline of the formula I or a pharmaceutically acceptable salts thereof and a suitable pharmaceutical carrier or excipient.

The ergoline derivatives of formula I and their salts induce less side effects than bromocriptine. They have a potent dopaminergic activity only on the central receptors when modified by extrapyramidal syndromes, such as in Parkinson's disease. They may be used alone or in association with other antiparkinson agents.

The orientative acute toxicity of the compounds I in rats is higher than 300 mg/Kg p.o..

The compounds are therefore indicated for use as antiparkinson agents.

The amount of active compound for this indication will, of course, be dependent on the subject being treated, the severity of the application, the manner of administration and the judgment of the prescribing physician.

However, an effective dosage is in the range of about 0.01 to about 5 mg, conveniently given in divided doses 1 to 5 times a day in unit dosage form containing from about 0.01 to about 2 mg of the compound or in sustained release form.

### ADMINISTRATION AND COMPOSITIONS

Administration of the active compound and salts described herein can be via any of the accepted modes of administration for antiparkinson agents.

The routes of administration include parenteral, oral, buccal, peroral, transdermal, intranosal or other suitable routes.

Depending on the intended route of administration, such compositions may be formulated in conventional manner or other pharmaceutical systems for delivery of the drug in a rate and extent needed for the intended therapeutical use.

The composition will include a conventional pharmaceutical carrier or excipient and an active compound of formula I or the pharmaceutically acceptable salts thereof and, in addition, may include other medicinal agents, pharmaceutical agents, carriers, adjuvants, etc.

For solid composition, conventional non toxic solid carriers include, for example, pharmaceutical grades of mannitol, lactose, starch, magnesium stearate, sodium saccharin, talcum, cellulose, glucose, sucrose, magnesium carbonate and the like may be used. Liquid pharmaceutically administerable compositions can, for example, be prepared by dissolving, dispersing, etc., an active compound as defined above and optional pharmaceutical adjuvant in a carrier, such as, for example, water, saline, aqueous dextrose, glycerol, ethanol and the like, to thereby form a solution or suspension.

The compound of example 1 is the preferred compound.

The following examples still further illustrate the invention without limiting it.

### Example 1

### 4-[(6-Methylergolen-Δ9,10-8β-yl)methyl]-piperidin-2,6-dione

To a solution of 5.3 g (C.015 mol) of (E)-3-[6-methylergolen Δ9,10-8β-yl)methyl]-ethyl acrylate and 0.25 g (0.01 mol) of sodium methoxide in 100 ml of 1,4-dioxane was added 2.62 g (0.02 mol) of ethoxycarbonylacetamide. The resulting suspension was refluxed for 4 hours. The solvent was removed, the residue was washed with water then crystallized from isopropyl alcohol affording 6.8 g of {3-[(6-methylergolen-Δ9,10-8β-yl)methyl]-4-amminocarbonyl}-diethylglutarate.

A solution of 10 g (0.021 mol) of this ester in 50 ml of ethanol and 42 ml of sodium hydroxide 1M was heated at 50°C for 30 minutes. This solution was acidified with 42 ml of 1m hydrochloric acid and then poured into icy water. The resulting precipitate was filtered off, washed with water and then with acetone, and dried to give 8.9 g of {3-[(6-methtylergolen-Δ9,10-8β-yl)methyl]-4-aminocarbonyl}-glutaric acid, as a mixture of diastereomers.

A solution of 7 g of the above mentioned glutaric acid derivative was dissolved in 150 ml of dimethylformamide was refluxed for 3 hours, then the solvent was evaporated off and the resulting product was crystallized from methanol yielding 5 g of 3-[(6-methylergolen-Δ9,10-8β-yl)methyl]-glutaric acid monoamide. To a suspension of 5.5 g (0.016 mol) of 3-[(6-methylergolen-Δ9,10-8β-yl)methyl]-glutaric acid monoamide in 150 ml of anhydrous acetonitrile was added portionwise g 3.9 (0.024 mol) of carbonyldiimidazole at room temperature. The resulting suspension was refluxed for 3 hours. The solvent was evaporated off and the residue was taken up in methylene chloride and washed with 10% of potassium carbonate solution. After drying, evaporation of the solvent and further crystallization from ethyl acetate 4.1 g of the title compound were obtained. m.p. 241°-243°C.

### Example 2

### 4-[(6-methylergolin-8β-yl)methyl]piperidin-2,6-dione

Operating as in Example 1 but employing (E)-3-[(6-methylergolin-8β-yl)methyl]-ethylacrylate in place of (E)-3-[(6-methylergolen-Δ9,10-8β-yl)methyl]-ethylacrylate, the title compound was obtained in 35% yield, m.p. 221°-223°C.

### Example 3

### 4-(6-methylergolin-8β-yl)-piperidin-2,6-dione

Operating as in Example 1 but employing (E)-3-(6-methylergolin-8β-yl)-ethylacrylate in place of (E)-3-[(6-methylergolen-Δ9,10-8β-yl)methyl]-ethylacrylate, the title compound was obtained in 25% yield, m.p. 197°-201°C.

### Example 4

### 4-[(6-Propylergolen-Δ9,10-8β-yl)methyl]-piperidin-2,6-dione

Operating as in Example 1, but employing (E)-3-[(6-propylergolen-Δ9,10-8β-yl)methyl]-ethylacrylate in place of (E)-[(6-methylergolen-Δ9,10-8β-yl)methyl]-ethylacrylate, the title compound was obtained in 10% yield, m.p. 157°-159°C.

### Example 5

### 1-Methyl-4-[(6-methylergolen-Δ9,10-8β-yl)methyl]-piperidin-2,6-dione

Operating as in Example 1, but employing ethoxycarbonyl-N-methyl acetamide in place of ethoxycarbonyl acetamide, the title compound was obtained in 32% yield, m.p. 181°-183°C.

### Example 6

### 4-[(6-Methylergolen-Δ9,10-8α-yl)methyl]-piperidin-2-6-dione

Operating as in Example 1, but employing (E)-3[(6-methylergolen-Δ9,10-8α-yl)methyl] -ethylacrylate in place of (E)-3-[(6-methylergolen- 9,10-8β-yl)methyl] -ethylacrylate, the title compound was obtained in 10% yield, m.p. 192°-195°C.

### Example 7

### 1-Phenyl-4-[(6-methylergolen-Δ9,10-8β-yl)methyl]-piperidin-2,6-dione

Operating as in example 1, but employing ethoxycarbonyl-N-phenylacetamide in place of ethoxycarbonylacetamide, the title compound was obtained in 30% yield, m.p. 223°-225°C.

### Example 8

### 4-(6-Methylergolen-Δ9,10-8β-yl)ethyl]-piperidin-2,6-dione

Operating as in example 1, but employing (E)-3[(6-methylergolen-Δ9,10-8α-yl)ethyl]-ethylacrylate in place of (E)-3-[(6-methylergolen-Δ 9,10-8β-yl)methyl]ethylacrylate, the title compound was obtained in 23% yield, m.p. 171°-173°C.

### Example 9

### 4-[(1-Ethyl-6-methylergolen-Δ9,10-8β-yl)methyl]-piperazin-2,6-dione

Operating as in example 1, but employing (E)-3[(1-ethyl-6-methylergolen-Δ9,10-8β-yl)methyl]-ethylacrylate in place of (E)-3-[(6-methylergolen-Δ9,10-8β-yl)methyl]ethylacrylate, the title compound was obtained in 20% yield, m.p. 141°-145°C.

### Example 10

### 4-[(2-Thiomethyl-6-methylergolen-Δ9,10-8β-yl)methyl]-piperidin-2,6-dione

Operating as in example 1, but employing (E)-3-[(2-thiomethyl-6-methylergolen-Δ9,10-8β-yl)methyl]-ethlacrylate, in place of (E)-[(6-methylergolen-Δ9,10-8β-yl)methyl]-ethylacrylate, the title compound was obtaines 27% yield, m.p. 181°-183°C.

## Claims

1. A compound of formula I 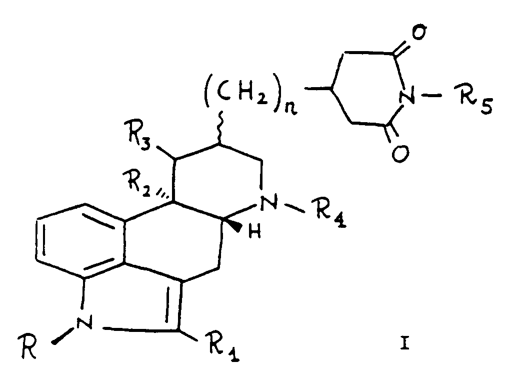 wherein R represents a hydrogen atom or a C₁-C₄ alkyl group; R₁ represents a hydrogen, a halogen atom, a methyl group, a phenylthio group or an alkylthio group having from 1 to 4 carbon atoms; R₂ represents a hydrogen atom or a methoxy group; R₃ represents a hydrogen atom or R₂ and R₃ together represent a chemical bond; R₄ represents a hydrocarbon group having from 1 to 4 carbon atoms; R₅ represents a hydrogen atom, an alkyl group having from 1 to 4 carbon atoms or a phenyl group and n is 0, 1 or 2 or a pharmaceutically acceptable salt thereof.

2. A compound according to claim 1 wherein R is a hydrogen atom or a methyl group.

3. A compound according to claim 1 or claim 2 wherein R₄ is a methyl group.

4. A compound according to any preceding claim wherein R₁ is a hydrogen, chlorine or bromine atom.

5. A compound according to any preceding claim wherein the substituent at the 8-position attached by the wavy line bond is beta-configuration.

6. A compound according to any preceding claim for use in treating diseases of the central nervous system.

7. A process for preparing a compound of formula I as defined in claim 1, which process comprises reacting a compound of formula II 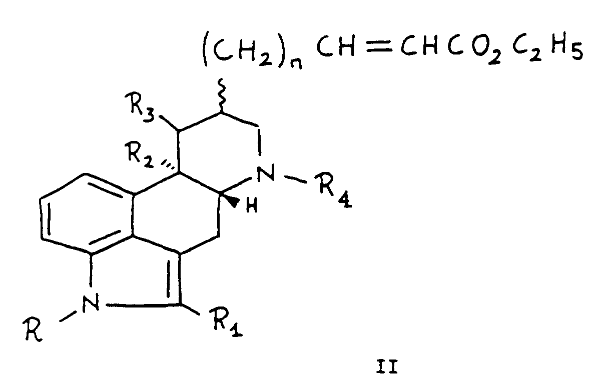 wherein R, R₁, R₂, R₄ and n are as defined in claim 1 with a compound of the formula III: 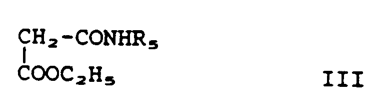 wherein R₅ is as defined in claim 1, saponificating and decarboxylating the resultant compound of formula IV: 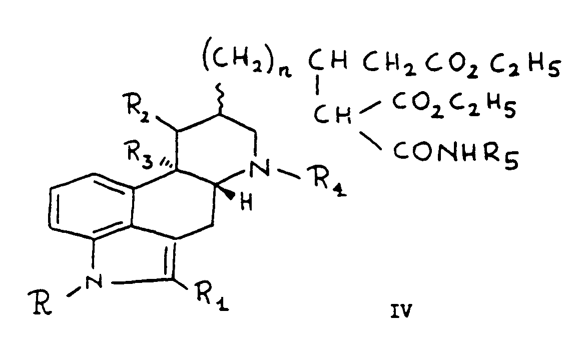 wherein R, R₁,R₂, R₃, R₄, R₅ and n are as defined above and cyclising the resultant compounds of formula V: 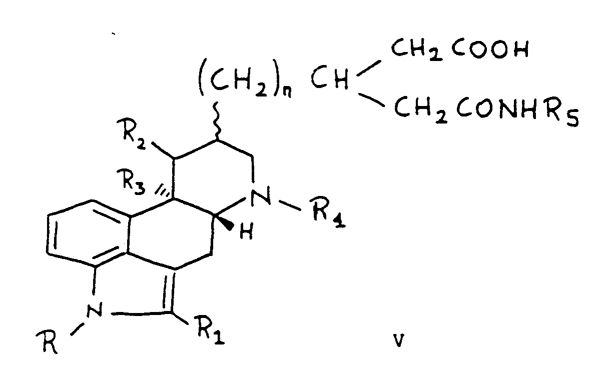 wherein R, R₁, R₂, R₃, R₄, R₅ and n are as defined above to give the desired compounds of formula I.

8. A process for preparing a compound of formula I as defined in claim 1 which comprises the step of cyclising a compound of formula V: 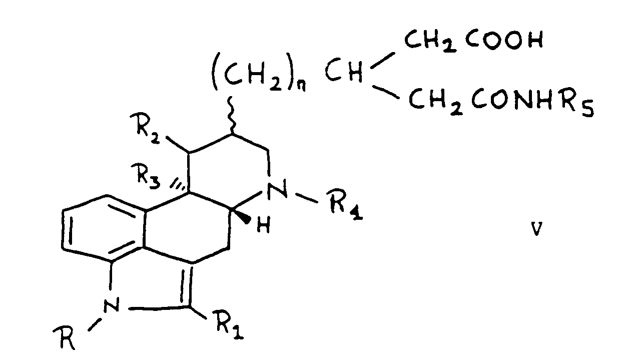 wherein R, R₁, R₂, R₃, R₄, R₅ and n are as defined in claim 1.

9. A pharmaceutical composition comprising an effective amount of a compound of the formula I as defined in claim 1 and a pharmaceutically acceptable diluent or carrier.

## Patentansprüche

1. Eine Verbindung der Formel I 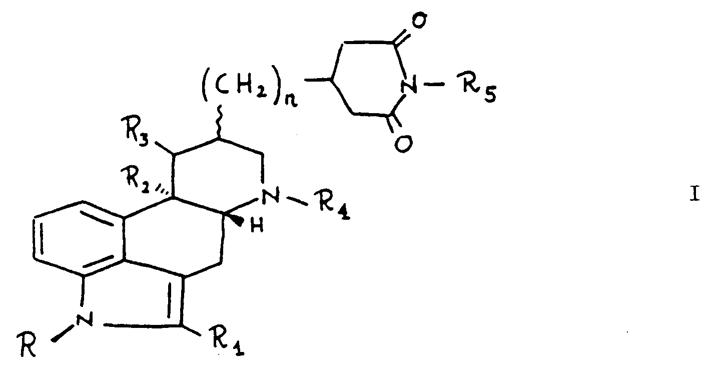 worin R ein Wasserstoffatom oder eine C₁-C₄ Alkylgruppe darstellt; R₁ ein Wasserstoffatom, ein Halogenatom, eine Methylgruppe, eine Phenylthiogruppe oder eine Alkylthiogruppe mit 1 bis 4 Kohlenstoffatomen darstellt; R₂ ein Wasserstoffatom oder eine Methoxygruppe darstellt; R₃ ein Wasserstoffatom darstellt oder R₂ und R₃ gemeinsam eine chemische Bindung darstellen; R₄ eine Kohlenwasserstoffgruppe mit 1 bis 4 Kohlenstoffatomen darstellt; R₅ ein Wasserstoffatom, eine Alkylgruppe mit 1 bis 4 Kohlenstoffatomen oder eine Phenylgruppe darstellt und n 0, 1 oder 2 ist oder ein pharmazeutisch annehmbares Salz derselben.

2. Eine Verbindung gemäss Patentanspruch 1, dadurch gekennzeichnet, dass R ein Wasserstoffatom oder eine Methylgruppe darstellt.

3. Eine Verbindung gemäss den Patentansprüchen 1 oder 2, dadurch gekennzeichnet, dass R₄ eine Methylgruppe darstellt.

4. Eine Verbindung gemäss einem der obengenannten Patentansprüche, dadurch gekennzeichnet, dass R₁ ein Wasserstoff-, ein Chlor- oder ein Bromatom darstellt.

5. Eine Verbindung gemäss einem der obengenannten Patentansprüche, dadurch gekennzeichnet, dass der an der Position 8 mittels einer welligen Verbindungslinie fixierte Substituent eine β-Konfiguration hat.

6. Eine Verbindung gemäss einem der obengenannten Patentansprüche, dadurch gekennzeichnet, dass sie zur Behandlung von Krankheiten des zentralen Nervensystems angewendet wird.

7. Ein Verfahren zur Herstellung einer wie im Patentanspruch 1 definierten Verbindung der Formel I, dadurch gekennzeichnet, dass es die Reaktion einer Verbindung der Formel II 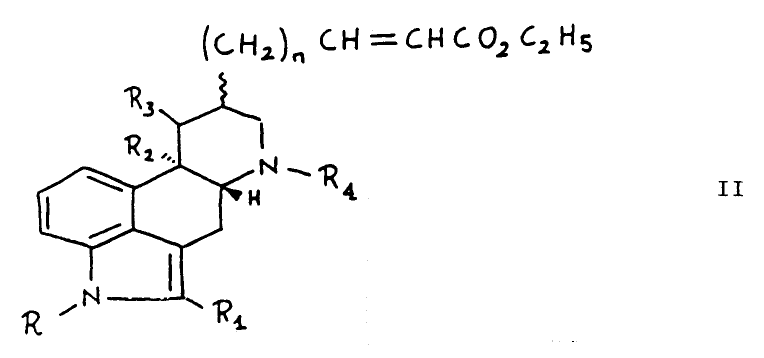 worin R, R₁, R₂, R₄ und n die im Patentanspruch 1 angegebenen Bedeutungen haben, mit einer Verbindung der Formel III: 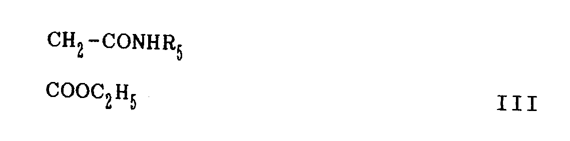 worin R₅ die im Patentanspruch 1 genannte Bedeutung hat, die Verseifung und die Dekarboxylierung der sich ergebenden Verbindung mit der Formel IV: 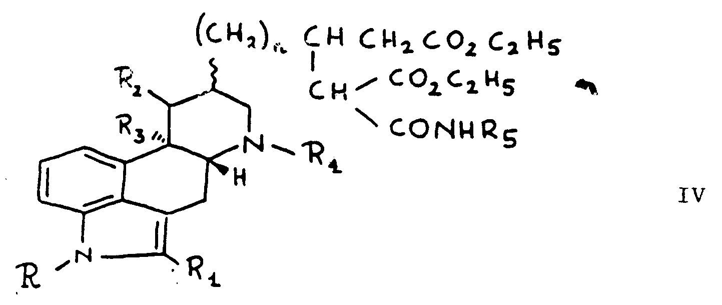 worin R, R₁, R₂, R₃, R₄, R₅ und n die obengenannten Bedeutungen haben, sowie die Zyklisierung der sich ergebenden Verbindungen mit der Formel V umfasst 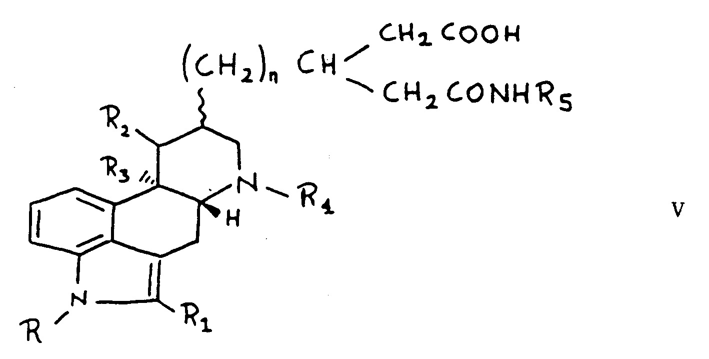 worin R, R₁, R₂, R₃, R₄, R₅ und n die obengenannten Bedeutungen haben, um die gewünschten Verbindungen der Formel I zu erhalten.

8. Ein Verfahren zur Herstellung einer wie im Patentanspruch 1 definierten Verbindung der Formel I, dadurch gekennzeichnet, dass es die Zyklisierungsstufe einer Verbindung der Formel V: 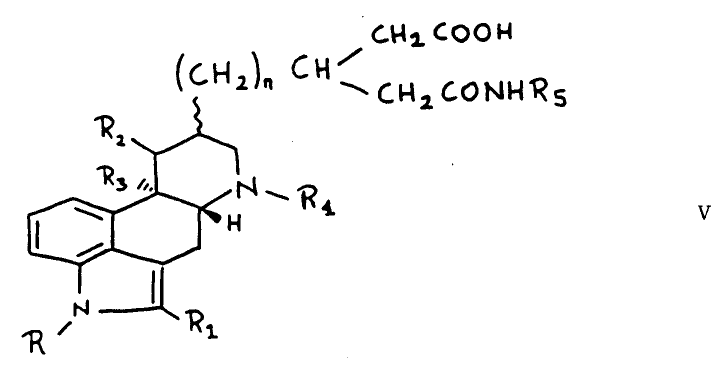 einschliesst, worin R, R₁, R₂, R₃, R₄, R₅ und n die im Patentanspruch 1 genannten Bedeutungen haben.

9. Eine pharmazeutische Zusammensetzung, dadurch gekennzeichnet, dass sie eine wirksame Menge einer so wie im Patentanspruch 1 definierten Verbindung der Formel I sowie einen pharmazeutisch annehmbaren Verdünner oder Träger enthält.

## Revendications

1. Composé représenté par la formule I 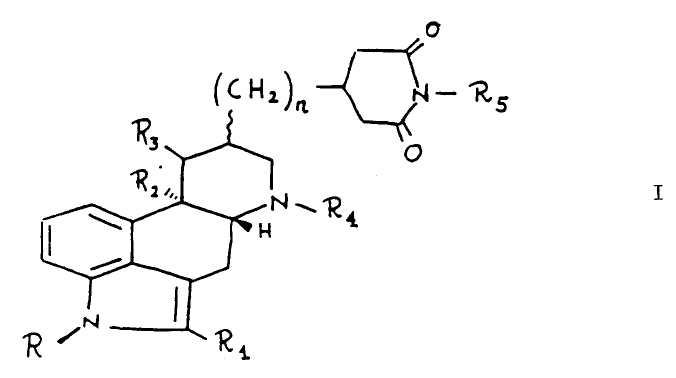 dans laquelle R représente un atome d'hydrogène ou un groupe C₁-C₄ alcoyle; R₁ représente un atome d'hydrogène, un atome d'halogène, un groupe méthyle, un groupe phénylthio ou un groupe alcoylthio ayant de 1 à 4 atomes de carbone; R₂ représente un atome d'hydrogène ou un groupe méthoxy; R₃ représente un atome d'hydrogène ou R₂ et R₃ ensemble représentent une liaison chimique; R₄ représente un groupe hydrocarbone ayant de 1 à 4 atomes de carbone; R₅ représente un atome d'hydrogène, un groupe alcoyle ayant de 1 à 4 atomes de carbone ou un groupe phényle et n est 0, 1 ou 2 ou un de ses sels acceptable du point de vue pharmaceutique.

2. Composé selon la revendication 1, caractérisé par le fait que R repésente un atome d'hydrogène ou un groupe méthyle.

3. Composé selon les revendications 1 ou 2, caractérisé par le fait que R₄ représente un groupe méthyle.

4. Composé selon les revendications susmentionnées, caractérisé par le fait que R₁ représente un atome d'hydrogène, un atome de chlore ou un atome de brome.

5. Composé selon les revendications susmentionnées, caractérisé par le fait que le substituant fixé en position 8 par une liaison à ligne ondulée a une configuration β.

6. Composé selon les revendications susmentionnées, caractérisé par le fait que l'on l'emploie dans le traitement des maladies du système nerveux central.

7. Procédé de fabrication d'un composé de formule I tel que défini à la revendication 1, comprenant la réaction d'un composé représenté par la formule II 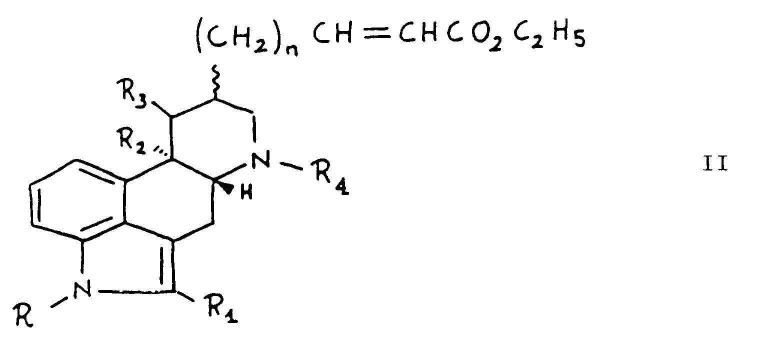 où R, R₁, R₂, R₄ et n ont les significations indiquées à la revendication 1, avec un composé de la formule III 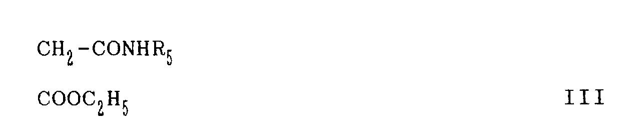 où R₅ a la signification indiquée à la revendication 1, la saponification et la décarboxylation du composé qui en résulte, ayant la formule IV 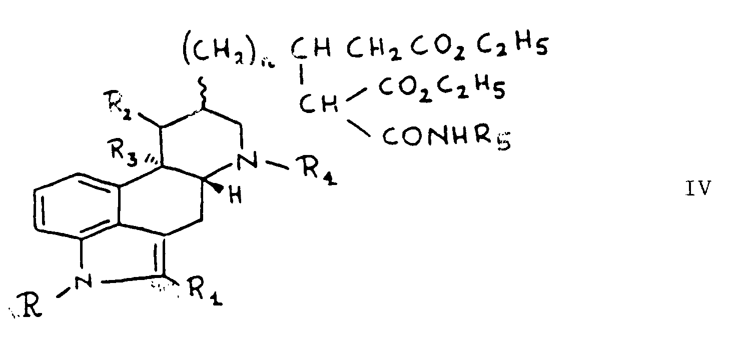 où R, R₁, R₂, R₃, R₄, R₅ et n ont les significations indiquées ci-dessus, ainsi que la cylicsation des composés qui en résultent, ayant la formule V 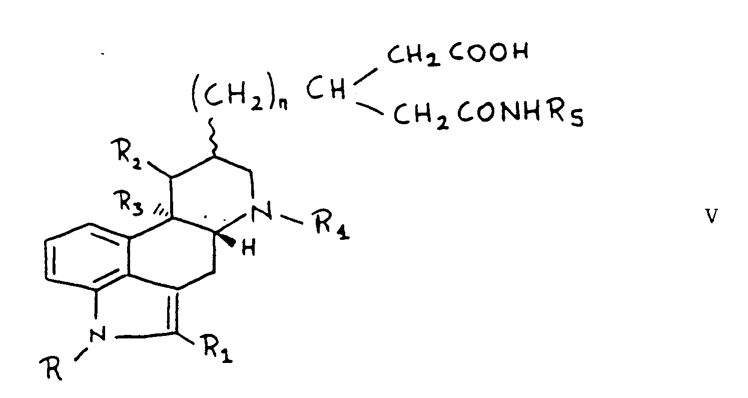 où R, R₁, R₂, R₃, R₄, R₅ et n ont les significations indiquées ci-dessus, pour obtenir les composés désirés de la formule I.

8. Procédé de fabrication d'un composé de formule I tel que défini à la revendication 1, caractérisé per le fait qu'il comprend l'étape de cyclisation d'un composé ayant la formule V 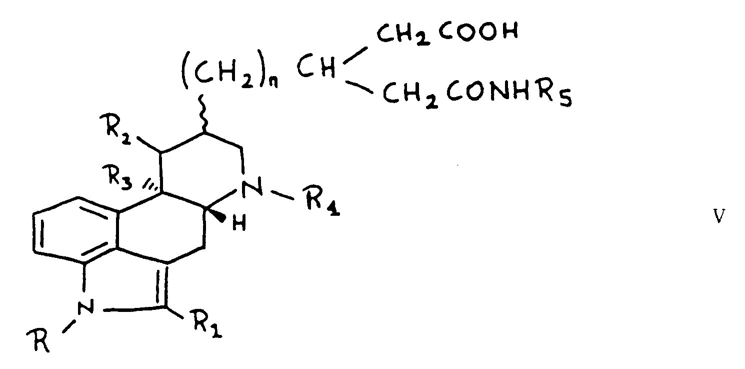 où R, R₁, R₂, R₃, R₄, R₅ et n ont les significations indiquées à la revendication 1.

9. Composition pharmaceutique, caractérisée par le fait qu'elle contient une quantité efficace d'un composé de la formule I tel que défini à la revendication 1 et un diluant ou un support acceptable du point de vue pharmaceutique.
